# EUROPEAN PATENT APPLICATION

(11) **EP 2 196 172 A1**
(43) Date of publication of application: **16.06.2010**
(21) Application number: 09178398.5
(22) Date of filing: 08.12.2009
(51) Int. Cl.: A61F 2/16

(54) **Intraocular implant with anti-glare coating**

(30) Priority: 10.12.2008 US 331656
(71) Applicant: Visioncare Ophthalmic Technologies, Inc., Saratoga, California 95070 (US)
(72) Inventor: Aharoni, Eli, 69400, Tel Aviv (IL); Dotan, Gideon, 56275, Yehud (IL)
(74) Representative: Frost, Alex John

(57) **Abstract**

An intraocular implant, including an implant body (250,402,512,700) adapted to have mounting haptics (252,404) attached thereto, the implant body including a generally cylindrical portion and at least one sealing element (260,406,408,518,520,716) mounted onto an end of the generally cylindrical portion, the intraocular implant also including at least one lens mounted within the implant body and an anti-glare coating layer (292,302,422,532,542,712) covering at least part of an outer area of the generally cylindrical portion, and being operative to prevent glare caused by light rays passing through the implant body from obstructing a user's vision.

## Description

### FIELD OF THE INVENTION

The present invention relates to optical implants generally and more particularly to anti-glare solutions for intraocular implants.

### BACKGROUND OF THE INVENTION

The following patent publications are believed to represent the current state of the art:
U.S. Patent Nos.: 5,628,794; 5,169,597; 6,632,887; 6,613,088 and 6,280,471; and
U.S. Patent Publication Nos.: 2002/0149741 and 2003/0229303.

### SUMMARY OF THE INVENTION

The present invention seeks to provide an intraocular implant, including an implant body adapted to have mounting haptics attached thereto, the implant body including a generally cylindrical portion and at least one sealing element mounted onto an end of the generally cylindrical portion, the intraocular implant also including at least one lens mounted within the implant body and an anti-glare coating layer covering at least part of an outer area of the generally cylindrical portion, and being operative to prevent glare caused by light rays passing through the implant body from obstructing a user's vision.

In accordance with a preferred embodiment of the present invention the coating layer includes a metallic layer including a plurality of longitudinal grooves operative to prevent the formation of eddy currents in the coating layer. Preferably, the coating layer is formed of titanium.

In accordance with another preferred embodiment of the present invention, the anti-glare coating layer is opaque. Preferably, the coating layer is applied onto the generally cylindrical portion by a sputtering process.

In accordance with yet another preferred embodiment of the present invention the at least one lens has an optical surface having optical power, and an inner boundary of the anti-glare coating layer lies at an edge of the optical surface. Preferably, the anti-glare coating layer covers an outer circumference of the generally cylindrical portion and extends onto the at least one sealing element.

There is also provided in accordance with another preferred embodiment of the present invention an intraocular implant, including an implant body and at least one lens, the implant body being at least partially coated by a metallic coating having a plurality of grooves formed therein, the grooves being operative to prevent the formation of eddy currents in the coating.

In accordance with a preferred embodiment of the present invention the coating is an anti-glare coating, operative to prevent glare caused by light rays passing through the implant body from obstructing a user's vision. Preferably, the coating layer is formed of titanium. Additionally or alternatively, the coating layer is opaque.

In accordance with another preferred embodiment of the present invention the coating layer is applied onto the generally cylindrical portion by a sputtering process. Preferably, the at least one lens has an optical surface having optical power, and an inner boundary of the coating layer lies at an edge of the optical surface. Additionally or alternatively, the implant body includes a generally cylindrical portion and at least one sealing element, and the coating layer covers an outer circumference of the generally cylindrical portion and extends onto the at least one sealing element.

### BRIEF DESCRIPTION OF THE DRAWINGS

The present invention will be understood and appreciated more fully from the following detailed description, taken in conjunction with the drawings in which:
Figs. 1A and 1B are simplified sectional illustration of a field of view widening telescopic implant constructed and operative in accordance with two alternative preferred embodiments of the present invention;
Fig. 2 is a simplified partially sectional illustration of an implant forming part of an artificial vision system, the implant being constructed and operative in accordance with another preferred embodiment of the present invention;
Figs. 3A and 3B are simplified sectional illustrations of an intraocular implant constructed and operative in accordance with two alternative preferred embodiments of the present invention; and
Fig. 4 is a simplified pictorial illustration of an implant body coated in accordance with yet a further preferred embodiment of the present invention.

### DETAILED DESCRIPTION OF PREFERRED EMBODIMENTS

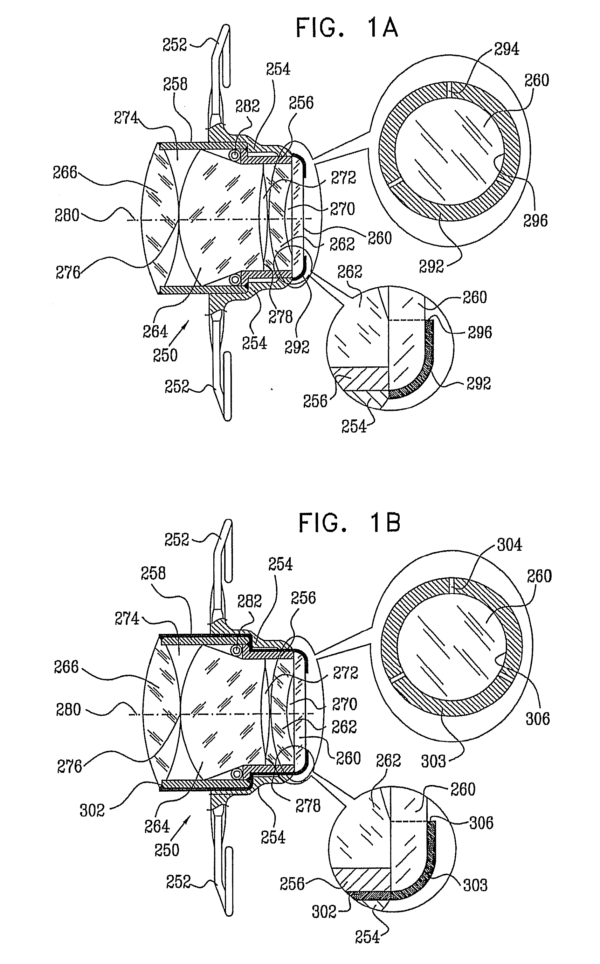

Reference is now made to Figs. 1A and 1B, which are simplified sectional illustration of a field of view widening telescopic implant constructed and operative in accordance with two alternative preferred embodiments of the present invention.

As seen in Figs. 1A and 1B, the implant preferably comprises an implant body 250, which is supported by haptics 252 via a haptic mounting structure 254. The implant body 250 typically comprises mutually sealed forward and rearward cylindrical housing portions 256 and 258 respectively and a transparent forward window 260 sealing the forward cylindrical portion 256. Typically, the implant body 250 is formed of glass housing portions, which are sealed by glass laser welding.

Disposed rearwardly of the forward window 260 in forward cylindrical portion 256 is a negative lens 262. Fixed to negative lens 262 as a doublet is a magnification lens 264, which resides partially in the forward cylindrical housing portion 256 and partially in the rearward cylindrical housing portion 258. Disposed rearwardly of the magnification lens 264 is a positive lens 266, which is mounted in sealing engagement with the rearward cylindrical housing portion 258 of implant body 250 and defines a rearward facing window.

Preferably, the negative lens 262 and the positive lens 266 include refractive and diffractive optical elements. Typically, the negative lens 262 and the positive lens 266 are coated with optical coatings.

It is an important feature of the present invention that the interior of the implant body 250 is sealed from the exterior thereof, so as to prevent liquids or vapors from entering the implant. It is also an important feature of the present invention that three air gaps, designated by reference numerals 270, 272 and 274, are provided to enhance refraction. The precision of the location of a contact point 276 between lenses 264 and 266 and of a peripheral contact area 278 between lenses 262 and 264 relative to an axis 280 is also of importance to maintain desired focus.

In accordance with a preferred embodiment of the present invention, a resilient O-ring 282 or other element having a similar function is provided to urge and retain lenses 264 and 266 in touching engagement at contact point 276.

Alternatively, the implant body may be formed of a single cylinder or of any suitable number of cylindrical portions. Furthermore, any suitable combination of any suitable number of lenses may be employed. Preferably, the haptics 252 are formed of a suitable polymer, the implant body 250 is formed of biocompatible glass and the forward window 260 and the lens 266 are formed of glass and are laser welded in sealing engagement with body 250.

Turning to Fig. 1A, it is seen that a metallic anti-glare coating layer 292 covers the outer circumference of forward window 260 of the implant body 250. The coating layer 292 is opaque, and therefore prevents light rays which pass through the transparent implant body 250 creating a glare which obstructs a user's vision.

As seen in the enlarged portion of Fig. 1A, the coating layer 292 includes a plurality of longitudinal grooves 294 which are operative to prevent the formation of eddy currents in the coating layer 292. The coating layer 292 is preferably formed of Titanium and is preferably applied to the implant body by a sputtering process.

It is appreciated that the intraocular implant 250 includes an optical surface having optical power, and that an inner boundary 296 of the coating layer 292 lies at an edge of the optical surface.

Turning now to Fig. 1B, it is seen that a metallic anti-glare coating layer 302 covers the outer circumference of the implant body 250 and extends onto forward window 260, as indicated by reference numeral 303. The coating layer 302 is opaque, and therefore prevents light rays which pass through the transparent implant body 250 creating a glare which obstructs a user's vision.

As seen in the enlarged portion of Fig. 1B, the coating layer 302 includes a plurality of longitudinal grooves 304 which are operative to prevent the formation of eddy currents in the coating layer 302. The coating layer 302 is preferably formed of Titanium and is preferably applied to the implant body by a sputtering process.

It is appreciated that the intraocular implant 250 includes an optical surface having optical power, and that an inner boundary 306 of portion 303 of the coating layer 302 lies at an edge of the optical surface.

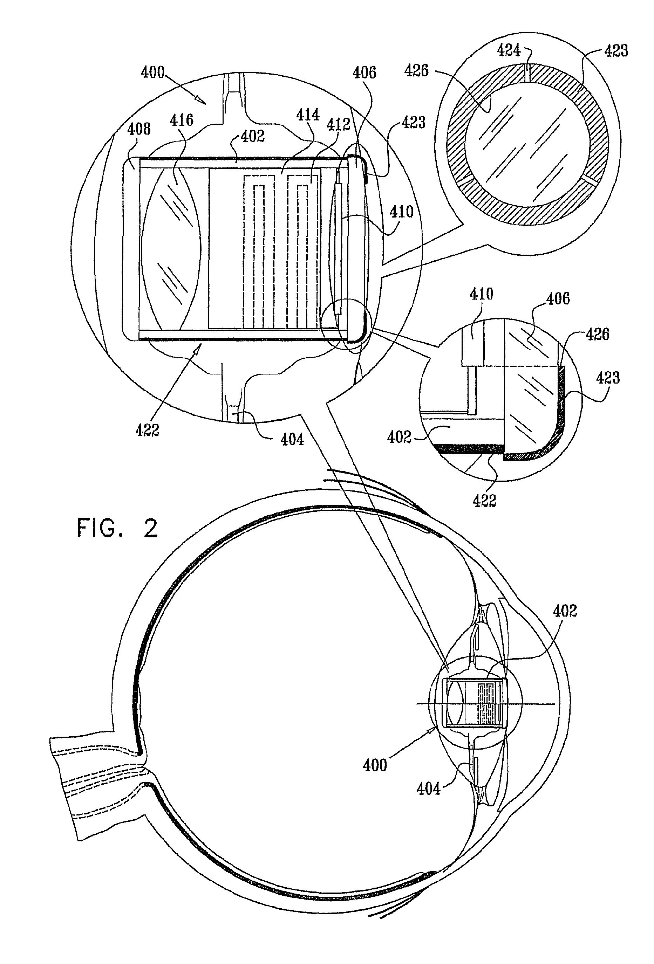

Reference is now made to Fig. 2, which is a simplified partially sectional illustration of an implant forming part of an artificial vision system, the implant being constructed and operative in accordance with another preferred embodiment of the present invention.

Fig. 2 illustrates an intraocular implant 400 which preferably forms part of an artificial vision system, such as that described in Applicants' U.S. Patent Application Ser. No. 10/489,388, the disclosure of which is hereby incorporated by reference. The intraocular implant 400 includes an intraocular implant body 402 having mounting haptics 404. Hermetically sealed to implant body 402 are a front sealing plate 406 and a back sealing plate 408. Back sealing plate 408 is transparent. An internal imaging device (not shown) is preferably mounted on an outside surface of front sealing plate 406. Capsules of this type are described in applicants' U.S. patent application Ser. No. 09/678,559, filed Oct. 3, 2000 and entitled "TELESCOPIC INTRAOCULAR LENS", which is a divisional application of U.S. patent application Ser. No. 09/222,330, filed Dec. 29, 1998 and entitled ""TELESCOPIC INTRAOCULAR LENS", subsequently abandoned, and U.S. patent application Ser. No. 09/721,916, filed Nov. 27, 2000 and entitled "TELESCOPIC INTRAOCULAR LENS", the disclosures of which are hereby incorporated by reference.

Preferably disposed within implant 400 is an electronic circuit and display assembly, which preferably includes electronic display 410 which is coupled to electronic circuitry 412, preferably including a wireless receiver for image data. Display 410 is arranged to lie generally parallel to front sealing plate 406, while electronic circuitry 412 is preferably embodied on a flexible circuit board 414 which is arranged to lie in a cylindrical configuration, peripherally of the optical path between display 410 and back sealing plate 408, so as not to interfere with the optical pathway between the display 410, focusing optics here shown as a lens 416, and the user's retina. It is appreciated that the focusing optics may also comprise multiple lenses.

As seen in Fig. 2, a metallic anti-glare coating layer 422 covers the outer circumference of the implant body 402, and extends onto front sealing plate 406 as indicated by reference numeral 423. The coating layer 422 is opaque, and therefore prevents light rays which pass through the transparent implant body 402 creating a glare which obstructs a user's vision.

As seen in the enlarged portion of Fig. 2, the coating layer 422 includes a plurality of longitudinal grooves 424 which are operative to prevent the formation of eddy currents in the coating layer 422. The coating layer 422 is preferably formed of Titanium and is preferably applied to the implant body by a sputtering process.

It is appreciated that the intraocular implant 400 includes an optical surface having optical power, and that an inner boundary 426 of portion 423 of the coating layer 422 lies at an edge of the optical surface.

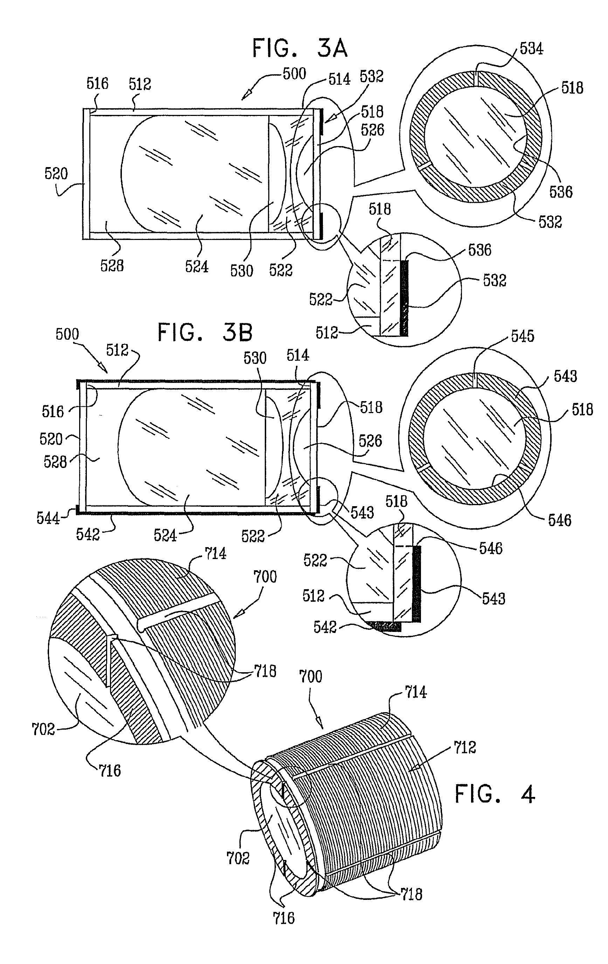

Reference is now made to Figs. 3A and 3B, which are simplified sectional illustrations of an intraocular implant constructed and operative in accordance with two alternative preferred embodiments of the present invention.

Figs. 3A and 3B illustrate a telescope 500, suitable for connection to haptics (not shown) for implantation in a human eye. The telescope 500 comprises a telescope body 512, typically of circular cylindrical configuration and formed of glass. Alternatively, the telescope body may be formed of other non-porous bio-compatible materials or may be formed of other materials and be coated with a suitable non-porous bio-compatible material.

Sealed to anterior and posterior ends 514 and 516 of the telescope body 512 are respective windows 518 and 520 which preferably do not have optical power. Mounted within telescope body 512 intermediate windows 518 and 520 are forward and rearward lenses, 522 and 524. Preferably air gaps 526 and 528 are defined between lenses 522 and 524 and respective windows 518 and 520 and an air gap 530 is defined between lenses 522 and 524.

Turning to Fig. 3A, it is seen that a metallic anti-glare coating layer 532 covers the outer circumference of forward window 518 of the implant body 512. The coating layer 532 is opaque, and therefore prevents light rays which pass through the transparent implant body 512 creating a glare which obstructs a user's vision.

As seen in the enlarged portion of Fig. 3A, the coating layer 532 includes a plurality of longitudinal grooves 534 which are operative to prevent the formation of eddy currents in the coating layer 532. The coating layer 532 is preferably formed of Titanium and is preferably applied to the implant body by a sputtering process.

It is appreciated that the intraocular implant 500 includes an optical surface having optical power, and that an inner boundary 536 of the coating layer 532 lies at an edge of the optical surface.

Turning now to Fig. 3B, it is seen that a metallic anti-glare coating layer 542 covers the outer circumference of the implant body 512 and extends onto forward window 518 and onto rearward window 520, as indicated by respective reference numerals 543 and 544. The coating layer 542 is opaque, and therefore prevents light rays which pass through the transparent implant body 512 creating a glare which obstructs a user's vision.

As seen in the enlarged portion of Fig. 3B, the coating layer 542 includes a plurality of longitudinal grooves 545 which are operative to prevent the formation of eddy currents in the coating layer 542. The coating layer 542 is preferably formed of Titanium and is preferably applied to the implant body by a sputtering process.

It is appreciated that the intraocular implant 500 includes an optical surface having optical power, and that an inner boundary 546 of portion 543 of the coating layer 542 lies at an edge of the optical surface.

Reference is now made to Fig. 4, which is a simplified pictorial illustration of an implant body coated in accordance with yet a further preferred embodiment of the present invention, the implant body being of the type described hereinabove with reference to any of Figs. 1A-3B.

As seen in Fig. 4, an implant body 700 has a generally circular cylindrical configuration, and typically has one or more lenses (not shown) disposed therein. The implant body 700 is typically sealed in the front and back by lenses and/or windows 702.

A metallic anti-glare coating layer 712 covers the outer circumference of the implant body 700 as indicated by reference numeral 714, and extends onto the front and rear sealing elements of the implant body 700, which may be windows or lenses, as indicated by reference numeral 716. The coating layer 712 is opaque, and therefore prevents light rays which pass through the transparent implant body 700 creating a glare which obstructs a user's vision. The coating layer 712 includes a plurality of longitudinal grooves 718, which extend along portions 714 and 716 thereof, and which are operative to prevent the formation of eddy currents in the coating layer 712. The coating layer 712 is preferably formed of Titanium and is preferably applied to the implant body by a sputtering process.

It will be appreciated by persons skilled in the art that the present invention is not limited by what has been particularly shown and described hereinabove. Rather the scope of the present invention includes both combinations and subcombinations of the various features described hereinabove as well as variations and modifications which would occur to persons skilled in the art upon reading the specification and which are not in the prior art.

## Claims

1. An intraocular implant, comprising:
an implant body adapted to have mounting haptics attached thereto, including:
a generally cylindrical portion; and
at least one sealing element mounted onto an end of said generally cylindrical portion;
at least one lens mounted within said implant body; and
an anti-glare coating layer covering at least part of an outer area of said generally cylindrical portion, and being operative to prevent glare caused by light rays passing through said implant body from obstructing a user's vision.

2. An intraocular implant according to claim 1 and wherein said coating layer comprises a metallic layer including a plurality of longitudinal grooves operative to prevent the formation of eddy currents in said coating layer.

3. An intraocular implant according to claim 2 and wherein said coating layer is formed of titanium.

4. An intraocular implant according to claim 1 and wherein said coating layer is opaque.

5. An intraocular implant according to claim 1 and wherein said coating layer is applied onto said generally cylindrical portion by a sputtering process.

6. An intraocular implant according to claim 1 and wherein said at least one lens has an optical surface having optical power, and an inner boundary of said anti-glare coating layer lies at an edge of said optical surface.

7. An intraocular implant according to claim 1 and wherein said anti-glare coating layer covers an outer circumference of said generally cylindrical portion and extends onto said at least one sealing element.

8. An intraocular implant, comprising an implant body and at least one lens, said implant body being at least partially coated by a metallic coating having a plurality of grooves formed therein, said grooves being operative to prevent the formation of eddy currents in said coating.

9. An intraocular implant according to claim 8 and wherein said coating is an anti-glare coating, operative to prevent glare caused by light rays passing through said implant body from obstructing a user's vision.

10. An intraocular implant according to claim 8 and wherein said coating layer is formed of titanium.

11. An intraocular implant according to claim 8 and wherein said coating layer is opaque.

12. An intraocular implant according to claim 8 and wherein said coating layer is applied onto said generally cylindrical portion by a sputtering process.

13. An intraocular implant according to claim 8 and wherein said at least one lens has an optical surface having optical power, and an inner boundary of said coating layer lies at an edge of said optical surface.

14. An intraocular implant according to claim 8 and wherein said implant body comprises a generally cylindrical portion and at least one sealing element, and said coating layer covers an outer circumference of said generally cylindrical portion and extends onto said at least one sealing element.
